# EUROPEAN PATENT SPECIFICATION

(11) **EP 3 456 264 B1**
(45) Date of publication and mention of the grant of the patent: **10.03.2021**
(21) Application number: 17796330.3
(22) Date of filing: 08.05.2017
(51) Int. Cl.: G16H 10/60, G16H 30/40, A61B 8/00, G16H 40/63

(54) **PORTABLE ULTRASONIC DIAGNOSTIC SYSTEM PROVIDING GUIDING ULTRASONIC IMAGE**
TRAGBARES ULTRASCHALLDIAGNOSESYSTEM MIT BEREITSTELLUNG VON ULTRASCHALLBILDFÜHRUNG
SYSTÈME DE DIAGNOSTIC ÉCHOGRAPHIQUE PORTABLE PRODUISANT UNE IMAGE ULTRASONORE DE GUIDAGE

(30) Priority: 10.05.2016 KR 20160057024
(43) Date of publication of application: 20.03.2019
(73) Proprietor: Healcerion Co., Ltd., Seoul 08376 (KR)
(72) Inventor: RYU, Jeong Won, Seoul 06768 (KR); CHOUNG, You Chan, Seoul 03427 (KR)
(74) Representative: Brann AB
(86) International application number: PCT/KR2017/004744
(87) International publication number: WO 2017/196030

(56) References cited:
- JP-A- H06 274 582
- JP-A- 2002 136 512
- JP-A- 2006 326 003
- KR-A- 20100 053 071
- KR-A- 20130 084 467
- US-A- 5 938 607
- US-A1- 2014 121 489
- US-A1- 2014 153 358
- US-A1- 2016 022 247

## Description

### Technical Field

The present invention relates to an ultrasonic diagnostic system, and more particularly, to a potable ultrasonic diagnostic system which connects a portable ultrasonic diagnostic device and a portable terminal to each other through wireless communication and performs an ultrasonic diagnosis.

### Background Art

Ultrasonic diagnoses are generally used in the medical field for obtaining information on an inside of an object to be inspected due to noninvasive and nondestructive properties thereof. Since ultrasonic diagnoses can provide a high-resolution image of internal organs of an object to be inspected, to a practitioner without a surgical operation of directly incising and observing the object, ultrasonic diagnostic systems are very significantly used.

An ultrasonic diagnostic system is a system which obtains images concerning tomography of soft tissue or blood flows without invasion by emitting an ultrasonic signal from a body surface of an object to be inspected toward a target part in a body and extracting information from the reflected ultrasonic signal.

Such ultrasonic diagnostic systems are generally used for diagnosing a heart, abdominal organs, urinary organs, and genital organs due to a small size, a low price, a capability of displaying in real time, and high security thereof without radiation exposure of X-rays or the like in comparison to an X-ray inspection device, a computerized tomography (CT) scanner, a magnetic resonance image (MRI) scanner, a nuclear medicine inspection device, and the like.

Recently, attempts have been made to embody a portable ultrasonic diagnostic device and connect a portable terminal such as a smart phone and a tablet personal computer (PC) to the ultrasonic diagnostic device through wireless communications so as to perform ultrasonic diagnoses.
US 2016/022247 A1 discloses an ultrasound imaging apparatus (100) including a receiving unit (350), a storage unit (500), a display unit (600) and a controlling unit (400). The display unit (600) displays a diagnostic imaging on a first section based on the imaging signal and displays an image indicator indicating a target part of the object on a second section, and a controlling unit (400) synchronizes the diagnostic imaging with a reference image corresponding to the diagnostic imaging.

### Disclosure of Invention

### Technical Problem

In obtaining of a diagnostic image by using an ultrasonic diagnostic device, a skilled expert such as a general practitioner may easily obtain a diagnostic image but an unskilled user may have trouble in obtaining a desired diagnostic image. That is, when a diagnosis is performed by using an ultrasonic diagnostic device, to obtain an adequate ultrasonic image, it is necessary but difficult for an unskilled user to perform scanning at a precise angle.

In addition, due to freely movable properties, a portable ultrasonic diagnostic device may be generally used by an unskilled user and may be usefully used, out of clinics, in an actual practice, and additionally, weak health-care countries which lack of medical facilities or medical teams.

The present invention is directed to providing a portable ultrasonic diagnostic system capable of easily performing a diagnosis and helping obtaining a desired diagnostic image when an unskilled user performs a diagnosis by using a portable ultrasonic diagnostic device.

### Technical Solution

In accordance with an aspect of the present invention, there is provided a portable ultrasonic diagnostic system as defined by claim 1. Further advantageous embodiments of the present invention are defined by the dependent claims.

One aspect of the present invention provides a portable ultrasonic diagnostic system including a portable ultrasonic diagnostic device, an ultrasonic diagnostic application which is installed in a portable terminal, receives ultrasonic image data from the portable ultrasonic diagnostic device, and displays an ultrasonic image through a display screen of the portable terminal, and an ultrasonic image server which stores guiding ultrasonic images for each diagnosis location and diagnosed part. Here, the ultrasonic diagnostic application transmits position information of the portable terminal and diagnosed part information to the ultrasonic image server. Also, the ultrasonic image server provides guiding ultrasonic images of a diagnosis location and a diagnosed part corresponding to the position information and the diagnosed part information to the ultrasonic diagnostic application. Also, the ultrasonic diagnostic application displays the ultrasonic image with the guiding ultrasonic image provided from the ultrasonic image server.

The diagnosed part information may be set by a user in the ultrasonic diagnostic application.

The ultrasonic image server may receive recommendation information of a guiding ultrasonic image to be recommended among guiding ultrasonic images for each diagnosed part from an ultrasonic diagnostic application whose user is a general practitioner, may record a recommendation number for each guide image, and may provide a guiding ultrasonic image whose recommendation number is greatest among the guiding ultrasonic images of the corresponding diagnosis location and diagnosed part to the ultrasonic diagnostic application. The ultrasonic image server may provide a plurality of guiding ultrasonic images of the corresponding diagnosis location and diagnosed part to each of ultrasonic diagnostic applications of a plurality of users, may allow the users to select the guiding ultrasonic image to be displayed with the ultrasonic image through the ultrasonic diagnostic application, may record a selection number for each guide image according to selections of the users, and may provide a guiding ultrasonic image whose selection number is greatest among the guiding ultrasonic images of the corresponding diagnosis location and diagnosed part to the ultrasonic diagnostic application.

The ultrasonic image server may record a signal to ratio (SNR) for each guiding ultrasonic image and may provide a guiding ultrasonic image whose SNR is highest among the guiding ultrasonic images of the corresponding diagnosis location and diagnosed part to the ultrasonic diagnostic application.

The ultrasonic diagnostic application may transmit gender information or age information of an object to be diagnosed to the ultrasonic image server, and the ultrasonic image server may provide a guiding ultrasonic image corresponding to the gender information or the age information among the guiding ultrasonic images of the corresponding diagnosis location and diagnosed part to the ultrasonic diagnostic application.

### Advantageous Effects

According to the present invention, an unskilled user may easily perform a diagnosis by using a portable ultrasonic diagnostic device, may obtain a desired diagnostic image, and may obtain a more accurate diagnostic image by using a guiding ultrasonic image on which a diagnosis location is reflected.

### Brief Description of Drawings

FIG. 1 illustrates components of a portable ultrasonic diagnostic system according to one embodiment of the present invention.
FIG. 2 illustrates a screen of an ultrasonic diagnostic application according to one embodiment of the present invention.
FIG. 3 illustrates additional components of a portable ultrasonic diagnostic system according to one embodiment of the present invention.
FIG. 4 illustrates additional components of a portable ultrasonic diagnostic system according to another embodiment of the present invention.
FIG. 5 illustrates one example of a list of guiding ultrasonic images which are stored in an ultrasonic image server.

### Mode for Invention

Hereinafter, exemplary embodiments of the present invention will be described in detail with reference to the attached drawings. In the following description and the attached drawings, substantially like elements are referred to as like reference numerals such that a repetitive description will be omitted. In the description of the present invention, a detailed description on well-known functions or components of the related art will be omitted when it is deemed to obscure the essence of the present invention.

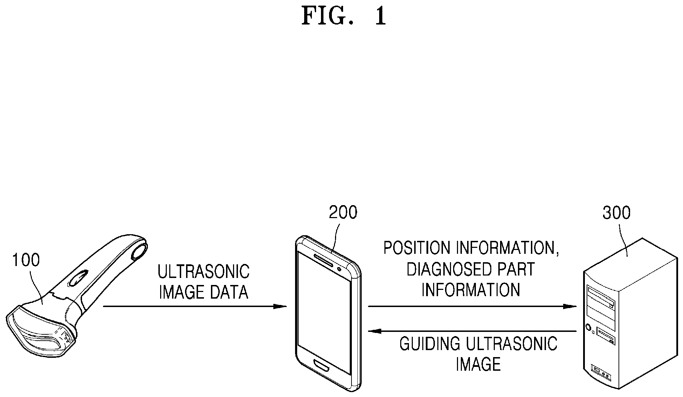

FIG. 1 illustrates components of a portable ultrasonic diagnostic system according to one embodiment of the present invention.

Referring to FIG. 1, a portable ultrasonic diagnostic system according to the embodiment includes an ultrasonic diagnostic device 100, a portable terminal 200, and an ultrasonic diagnostic application installed thereon, and an ultrasonic image server 300.

The portable ultrasonic diagnostic device 100 includes an ultrasonic probe, which transmits an ultrasonic signal to an object to be inspected and receives an ultrasonic echo signal reflected by the object and transmits ultrasonic image data (for example, scan line data or frame data) obtained from the ultrasonic echo signal to the portable terminal 200. The portable ultrasonic diagnostic device 100 includes a communication module for transmitting and receiving data with the portable terminal 200. Data transmission and reception may be performed between the portable ultrasonic diagnostic device 100 and the portable terminal 200 by using a wired or wireless method. As the wired communication method, a cable such as a USB cable and the like may be used. As the wireless communication method, Bluetooth, wireless USB, wireless LAN, WiFi, Zigbee, IrDA and the like may be used.

The portable terminal 200 is a mobile user terminal and includes any terminals which have an operating system and are capable of accessing the Internet and in which a variety of applications are installable. The portable terminal 200 may be, for example, a smart phone, a tablet PC, a laptop PC, and the like. In the portable terminal 200, an ultrasonic diagnostic application, which interworks with the portable ultrasonic diagnostic device 100 and performs an ultrasonic diagnostic function, is installed. Ultrasonic diagnostic application receives ultrasonic image data from the portable ultrasonic diagnostic device 100, converts the received ultrasonic image data into an ultrasonic image adequate for resolution of a display screen of the portable terminal 200, and displays the ultrasonic image through the display screen.

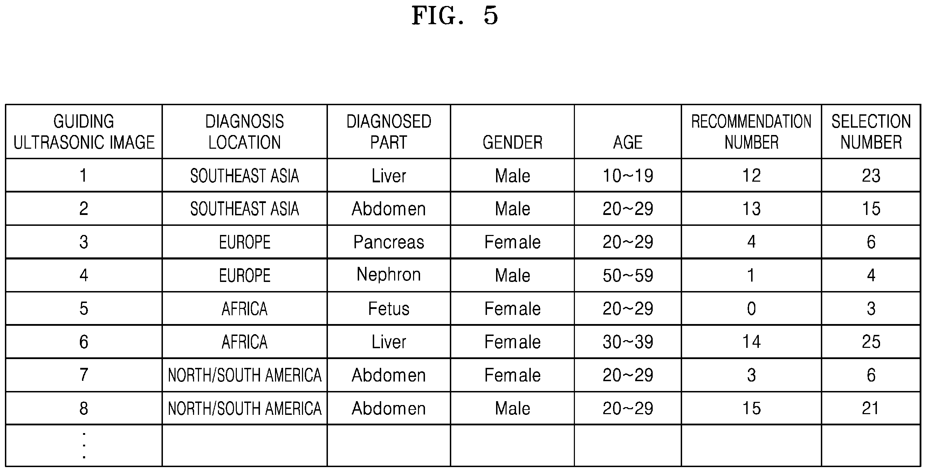

The ultrasonic image server 300 stores guiding ultrasonic images for each diagnosis location and diagnosed part. FIG. 5 illustrates one example of a list of guiding ultrasonic images which are stored in an ultrasonic image server. As shown in FIG. 5, diagnosis locations may be classified into, for example, Southeast Asia, Europe, Africa, North/South America, and the like, and diagnosed parts may include, for example, a liver, abdomen, pancreas, nephron, a fetus, and the like. The ultrasonic image server 300 may record, in addition to a diagnosis location and a diagnosed part for each guiding ultrasonic image, gender information and age information of the corresponding guiding ultrasonic image.

The guiding ultrasonic image is, for example, a so-called properly obtained ultrasonic image of the corresponding diagnosed part in the corresponding diagnosis location by an expert such as a general practitioner. To collect a guiding ultrasonic image, the ultrasonic image server 300 may collect ultrasonic images of each diagnosed part which are possessed by general practitioners in a variety of areas. According to an embodiment, when a user of the ultrasonic diagnostic application is an expert such as a general practitioner, authority to upload an ultrasonic image obtained using the ultrasonic diagnostic application on the ultrasonic image server 300 is given to the user such that guiding ultrasonic images are collected through the portable ultrasonic diagnostic device 100 and the ultrasonic diagnostic application of the portable terminal 200. Here, a diagnosis location may be obtained using position information of the portable terminal 200, and information such as a diagnosed part, a gender, an age, and the like may be information input by the user through the ultrasonic diagnostic application or information already included in an ultrasonic image.

The portable terminal 200 and the ultrasonic image server 300 are connected through a general wired or wireless network. When the user of the portable ultrasonic diagnostic device 100 selects of viewing a guiding ultrasonic image with setting a diagnosed part to be currently diagnosed through the ultrasonic diagnostic application of the portable terminal 200, the ultrasonic diagnostic application transmits a request for a guiding ultrasonic image including position information of the portable terminal 200 and diagnosed part information to the ultrasonic image server 300. Here, the position information of the portable terminal 200 is a information basically provided by the portable terminal 200 and may be obtained through, for example, a global position system (GPS), WiFi, or a base station. When the ultrasonic diagnostic application transmits the request for a guiding ultrasonic image to the ultrasonic image server 300, the ultrasonic diagnostic application may transmit gender information or age information of an object to be diagnosed. Here, the gender information or age information may be information input through the ultrasonic diagnostic application or may be information already included in an ultrasonic image being diagnosed.

The ultrasonic image server 300, which receives the request for a guiding ultrasonic image from the portable terminal 200, extracts, among guiding ultrasonic images, requested position information, a diagnosis location corresponding to diagnosed part information, and a guiding ultrasonic image corresponding to a diagnosis location, and provides the extracted guiding ultrasonic image to the ultrasonic diagnostic application through the wired or wireless communication network. When the request for a guiding ultrasonic image includes gender information or age information, the ultrasonic image server 300 may provide a guiding ultrasonic image corresponding to the requested gender or age among the guiding ultrasonic images of the corresponding diagnosis location and the diagnosed part.

The ultrasonic image server 300 may include several guiding ultrasonic images of each diagnosis location and diagnosed part. In this case, the ultrasonic image server 300 may provide one or more guiding ultrasonic images which are randomly selected among the guiding ultrasonic images of the corresponding diagnosis location and diagnosed part. According to an embodiment, the ultrasonic image server 300 may calculate and record a signal to ratio (SNR) for each guiding ultrasonic image, may provide a guiding ultrasonic image which has a highest SNR among guiding ultrasonic images of the corresponding diagnosis location and diagnosed part (according to circumstances, additionally, corresponding to a requested gender or age), or may provide several guiding ultrasonic images in a sequence of high SNR.

The ultrasonic diagnostic application displays a guiding ultrasonic image provided from the ultrasonic image server 300 with an ultrasonic image being currently diagnosed. Since the user may perform an ultrasonic diagnosis while comparing the ultrasonic image being currently diagnosed with the guiding ultrasonic image through the ultrasonic diagnostic application, the diagnosis may be easily performed and a desired diagnostic image may be more easily obtained.

In the embodiment of the present invention, storing guiding ultrasonic images classified for each diagnosis location and providing a guiding ultrasonic image in an area in which the user performs a diagnosis are in consideration of features of ultrasonic images which are different according to the diagnosis location since a race or a body shape generally varies according to an area. Accordingly, according to the embodiment of the present invention, a guiding ultrasonic image, on which a diagnosis location is reflected, may help obtaining a more accurate diagnostic image. For example, when an ultrasonic diagnosis is performed on a fetus in Africa, since an ultrasonic image of a fetus diagnosed (by a general practitioner) in Africa is used as a guiding ultrasonic image, it is possible to obtain a more accurate diagnostic image than when general ultrasonic images or guiding ultrasonic images which are diagnosed in other areas are used.

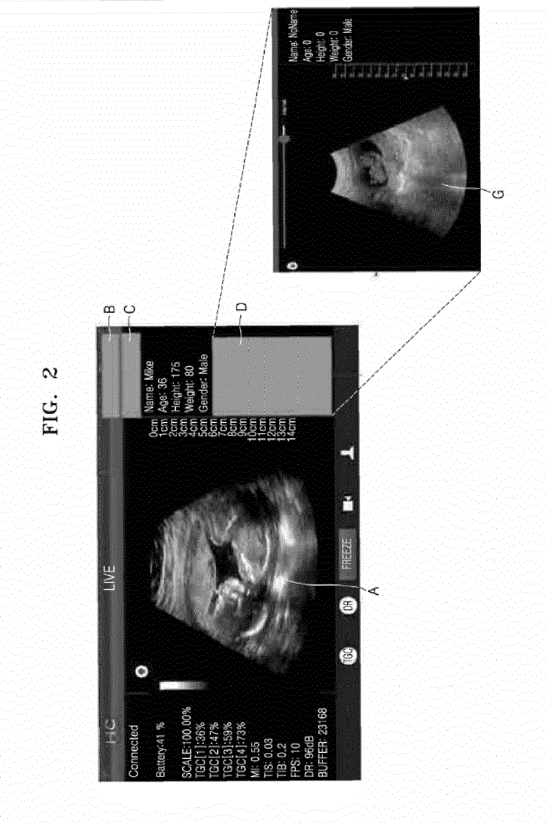

FIG. 2 illustrates a screen of the ultrasonic diagnostic application according to one embodiment of the present invention.

Screens of the ultrasonic diagnostic application according to the embodiment may include an area A in which an ultrasonic image being currently diagnosed is displayed, an area B in which a diagnosis location is displayed, an area C in which a diagnosed part is displayed, and an area D in which a guiding ultrasonic image is displayed. As shown in the area D in which a guiding ultrasonic image is displayed, a guiding ultrasonic image G may be displayed.

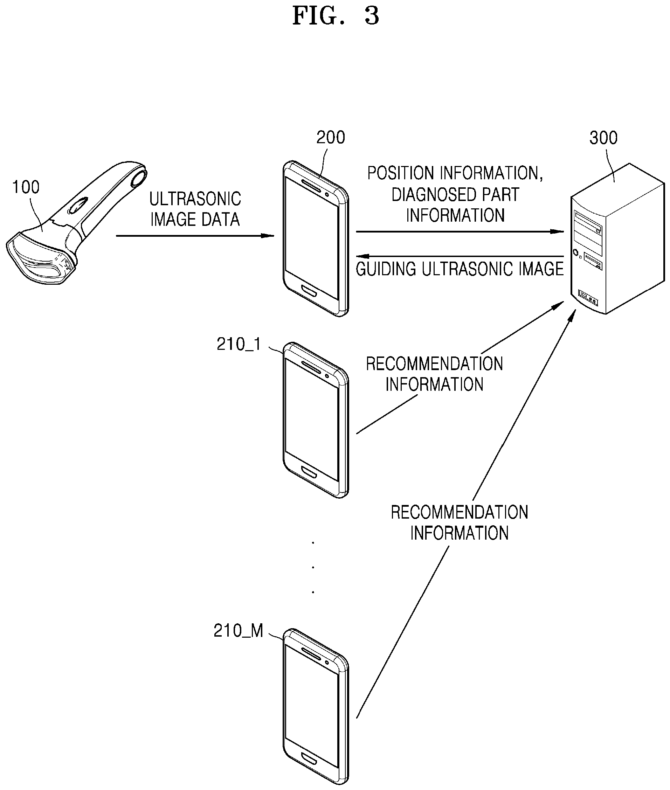

FIG. 3 illustrates additional components of a portable ultrasonic diagnostic system according to one embodiment of the present invention.

Referring to FIG. 3, the portable ultrasonic diagnostic system according to the embodiment further includes portable terminals 210_1 to 210_M whose users are experts, for example, general practitioners and ultrasonic diagnostic applications installed therein in the portable ultrasonic diagnostic system of FIG. 1.

The users of the portable terminals 210_1 to 210_M may access and refer to the ultrasonic image server 300 through the ultrasonic diagnostic application for guiding ultrasonic images for each diagnosed part and may recommend guiding ultrasonic images, which are determined by the users to be more adequate. Then, the ultrasonic diagnostic application may transmit recommendation information (that is, identification information of a recommended guiding ultrasonic image) to the ultrasonic image server 300, and the ultrasonic image server 300 may record the number of recommendations for each guide image as shown in FIG. 5 on the basis of recommendation information collected through the ultrasonic diagnostic application of each of the portable terminals 210_1 to 210_M.

When a guiding ultrasonic image is provided to the ultrasonic diagnostic application of the portable terminal 200, the ultrasonic image server 300 may provide a guiding ultrasonic image whose recommendation number is greatest or several guiding ultrasonic images in a sequence of higher recommendation number among guiding ultrasonic images of a corresponding diagnosis location and a diagnosed part (according to circumstances, additionally, corresponding to a requested gender or age).

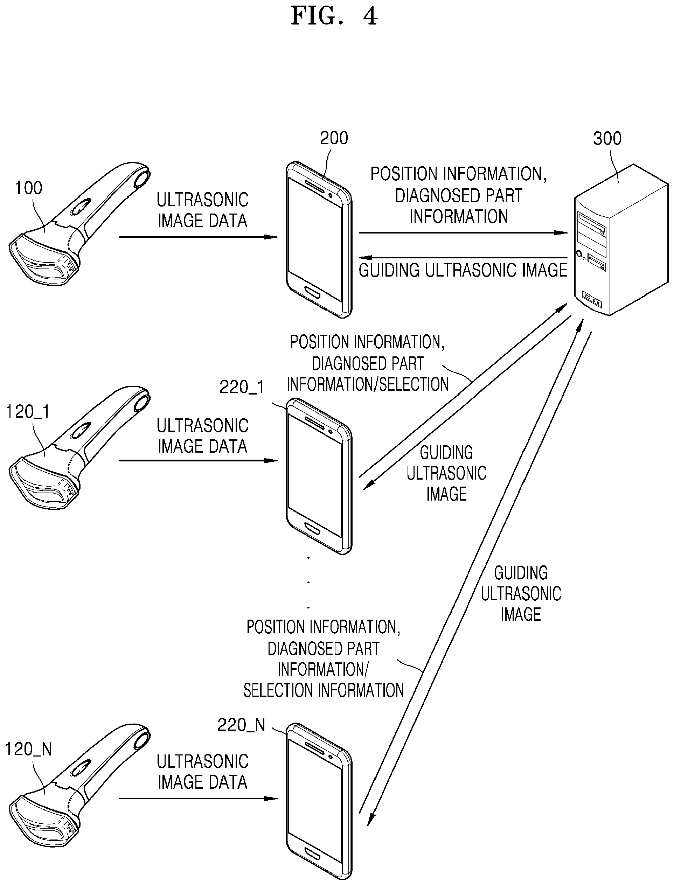

FIG. 4 illustrates additional components of a portable ultrasonic diagnostic system according to another embodiment of the present invention.

Referring to FIG. 4, the portable ultrasonic diagnostic system according to the embodiment further includes portable terminals 120_1 to 120 M of a plurality of users, portable terminals 220_1 to 220 N, and ultrasonic diagnostic applications installed therein in the portable ultrasonic diagnostic system of FIG. 1.

The ultrasonic diagnostic applications of the portable terminals 220_1 to 220 N, like the portable terminal 200, transmit a request for a guiding ultrasonic image to the ultrasonic image server 300 and display a guiding ultrasonic image provided by the ultrasonic image server 300. Here, the ultrasonic image server 300 may provide a plurality of guiding ultrasonic images of a corresponding diagnosis location and a diagnosed part to the ultrasonic diagnostic applications of the portable terminals 220_1 to 220 N. One of the users may select a guiding ultrasonic image to be displayed with an ultrasonic image being currently diagnosed through the ultrasonic diagnostic application, among the provided guiding ultrasonic images. Then, the ultrasonic diagnostic application may transmit selection information (that is, identification information of a selected guiding ultrasonic image) to the ultrasonic image server 300, and the ultrasonic image server 300 may record the number of selections for each guide image as shown in FIG. 5 on the basis of selection information collected through the ultrasonic diagnostic application of each of the portable terminals 220_1 to 220 N.

When a guiding ultrasonic image is provided to the ultrasonic diagnostic application of the portable terminal 200, the ultrasonic image server 300 may provide a guiding ultrasonic image whose selection number is greatest or several guiding ultrasonic images in a sequence of higher selection number among guiding ultrasonic images of a corresponding diagnosis location and a diagnosed part (according to circumstances, additionally, corresponding to a requested gender or age). The user of the portable terminal 200 may also select a guiding ultrasonic image, among several provided guiding ultrasonic images, to be displayed with an ultrasonic image being currently diagnosed, corresponding selection information may be transmitted to the ultrasonic image server 300 and may be reflected on a selection number.

Meanwhile, the above-described embodiments of the present invention may be written as a program, which is executable by a computer, and may be embodied in a universal digital computer which operates the program using a computer-readable recording medium. The computer-readable recording medium includes a storage medium such as a magnetic storage medium (for example, a read-only memory (ROM), a floppy disk, a hard disk, and the like) and an optical-readable medium (for example, a compact disc read-only memory (CD-ROM), a digital versatile disc (DVD), and the like).

The exemplary embodiments of the present invention have been described above. It will be understood by one of ordinary skill in the art that modifications may be made without departing from the essential features of the present invention. Therefore, the disclosed embodiments should be considered in a descriptive point of view not in a limitative one. The scope of the present invention is defined by the claims not by the above description, and it should be understood that all differences within the equivalent scope thereof are included in the present invention.

## Claims

1. A portable ultrasonic diagnostic system comprising:
a portable ultrasonic diagnostic device (100);
an ultrasonic diagnostic application installed in a portable terminal (200), wherein the ultrasound diagnostic application is configured to receive ultrasonic image data from the portable ultrasonic diagnostic device (100), and display an ultrasonic image through a display screen of the portable terminal (200); and
an ultrasonic image server (300) configured to store guiding ultrasonic images for various diagnosis locations and diagnosed parts,
wherein the ultrasonic diagnostic application in the portable terminal (200) is configured to transmit position information of the portable terminal (200) and diagnosed part information to the ultrasonic image server (300),
wherein the ultrasonic image server (300) is configured to provide guiding ultrasonic images of a diagnosis location and a diagnosed part corresponding to the position information of the portable terminal (200) and the diagnosed part information to the ultrasonic diagnostic application, and
wherein the ultrasonic diagnostic application in the portable terminal (200) is configured to display the ultrasonic image with a guiding ultrasonic image provided from the ultrasonic image server (300).

2. The portable ultrasonic diagnostic system of claim 1, wherein the diagnosed part information is set by a user in the ultrasonic diagnostic application.

3. The portable ultrasonic diagnostic system of claim 1, wherein the ultrasonic image server (300) is configured to receive recommendation information of a guiding ultrasonic image to be recommended among guiding ultrasonic images for each diagnosed part from an ultrasonic diagnostic application whose user is a general practitioner, record a recommendation number for each guide image, and provide a guiding ultrasonic image whose recommendation number is greatest among the guiding ultrasonic images of the corresponding diagnosis location and diagnosed part to the ultrasonic diagnostic application.

4. The portable ultrasonic diagnostic system of claim 1, wherein the ultrasonic image server (300) is configured to provide a plurality of guiding ultrasonic images of the corresponding diagnosis location and diagnosed part to each of ultrasonic diagnostic applications of a plurality of users, allow the users to select the guiding ultrasonic image to be displayed with the ultrasonic image through the ultrasonic diagnostic application, record a selection number for each guide image according to selections of the users, and provide a guiding ultrasonic image whose selection number is greatest among the guiding ultrasonic images of the corresponding diagnosis location and diagnosed part to the ultrasonic diagnostic application.

5. The portable ultrasonic diagnostic system of claim 1, wherein the ultrasonic image server (300) is configured to record a signal to ratio (SNR) for each guiding ultrasonic image and provide a guiding ultrasonic image whose SNR is highest among the guiding ultrasonic images of the corresponding diagnosis location and diagnosed part to the ultrasonic diagnostic application.

6. The portable ultrasonic diagnostic system of claim 1, wherein the ultrasonic diagnostic application is configured to transmit gender information or age information of an object to be diagnosed to the ultrasonic image server (300), and
wherein the ultrasonic image server (300) is configured to provide a guiding ultrasonic image corresponding to the gender information or the age information among the guiding ultrasonic images of the corresponding diagnosis location and diagnosed part to the ultrasonic diagnostic application.

## Patentansprüche

1. Tragbares Ultraschalldiagnosesystem, umfassend:
eine tragbare Ultraschalldiagnosevorrichtung (100);
eine Ultraschalldiagnoseanwendung, die auf einem tragbaren Endgerät (200) installiert ist, wobei die Ultraschalldiagnoseanwendung dazu konfiguriert ist, Ultraschallbilddaten von der tragbaren Ultraschalldiagnosevorrichtung (100) zu empfangen und ein Ultraschallbild durch einen Anzeigebildschirm des tragbaren Endgeräts (200) anzuzeigen; und
einen Ultraschallbildserver (300), der dazu konfiguriert ist, Ultraschallmusterbilder für verschiedene Diagnoseorte und diagnostizierte Teile zu speichern,
wobei die Ultraschalldiagnoseanwendung in dem tragbaren Endgerät (200) dazu konfiguriert ist, Positionsinformationen des tragbaren Endgeräts (200) und Informationen über einen diagnostizierten Teil zu dem Ultraschallbildserver (300) zu senden,
wobei der Ultraschallbildserver (300) dazu konfiguriert ist, Ultraschallmusterbilder eines Diagnoseorts und eines diagnostizierten Teils, die den Positionsinformationen des tragbaren Endgeräts (200) und den Informationen über den diagnostizierten Teil entsprechen, an die Ultraschalldiagnoseanwendung bereitzustellen, und
wobei die Ultraschalldiagnoseanwendung in dem tragbaren Endgerät (200) dazu konfiguriert ist, das Ultraschallbild mit einem von dem Ultraschallbildserver (300) bereitgestellten Ultraschallmusterbild anzuzeigen.

2. Tragbares Ultraschalldiagnosesystem nach Anspruch 1, wobei die Informationen über den diagnostizierten Teil durch einen Benutzer in der Ultraschalldiagnoseanwendung festgelegt werden.

3. Tragbares Ultraschalldiagnosesystem nach Anspruch 1, wobei der Ultraschallbildserver (300) dazu konfiguriert ist, Empfehlungsinformationen über ein Ultraschallmusterbild, das unter Ultraschallmusterbildern für jeden diagnostizierten Teil zu empfehlen ist, von einer Ultraschalldiagnoseanwendung, deren Benutzer ein Allgemeinarzt ist, zu empfangen, eine Empfehlungsnummer für jedes Musterbild aufzuzeichnen und ein Ultraschallmusterbild, dessen Empfehlungsnummer unter den Ultraschallmusterbildern des entsprechenden Diagnoseorts und diagnostizierten Teils am größten ist, an die Ultraschalldiagnoseanwendung bereitzustellen.

4. Tragbares Ultraschalldiagnosesystem nach Anspruch 1, wobei der Ultraschallbildserver (300) dazu konfiguriert ist, eine Vielzahl von Ultraschallmusterbildern des entsprechenden Diagnoseorts und diagnostizierten Teils an jede von Ultraschalldiagnoseanwendungen einer Vielzahl von Benutzern bereitzustellen, es den Benutzern zu ermöglichen, das Ultraschallmusterbild auszuwählen, das mit dem Ultraschallbild durch die Ultraschalldiagnoseanwendung anzuzeigen ist, eine Auswahlnummer für jedes Musterbild gemäß Auswahlen der Benutzer aufzuzeichnen und ein Ultraschallmusterbild, dessen Auswahlnummer unter den Ultraschallmusterbildern des entsprechenden Diagnoseorts und diagnostizierten Teils am größten ist, an die Ultraschalldiagnoseanwendung bereitzustellen.

5. Tragbares Ultraschalldiagnosesystem nach Anspruch 1, wobei der Ultraschallbildserver (300) dazu konfiguriert ist, ein Signal-Verhältnis (SNR) für jedes Ultraschallmusterbild aufzuzeichnen und ein Ultraschallmusterbild, dessen SNR unter den Ultraschallmusterbildern des entsprechenden Diagnoseorts und diagnostizierten Teils am höchsten ist, an die Ultraschalldiagnoseanwendung bereitzustellen.

6. Tragbares Ultraschalldiagnosesystem nach Anspruch 1, wobei die Ultraschalldiagnoseanwendung dazu konfiguriert ist, Geschlechtsinformationen oder Altersinformationen eines zu diagnostizierenden Objekts an den Ultraschallbildserver (300) zu senden, und
wobei der Ultraschallbildserver (300) dazu konfiguriert ist, ein Ultraschallmusterbild, das unter den Ultraschallmusterbildern des entsprechenden Diagnoseorts und diagnostizierten Teils der Geschlechtsinformationen oder der Altersinformationen entspricht, an die Ultraschalldiagnoseanwendung bereitzustellen.

## Revendications

1. Système de diagnostic échographique portable comprenant :
un dispositif de diagnostic échographique portable (100) ;
une application de diagnostic échographique installée dans un terminal portable (200), dans lequel l'application de diagnostic échographique est configurée pour recevoir des données d'image échographique à partir du dispositif de diagnostic échographique portable (100), et afficher une image échographique à travers un écran d'affichage du terminal portable (200) ; et
un serveur d'images échographiques (300) configuré pour stocker des images échographiques de guidage pour diverses localisations de diagnostic et parties diagnostiquées,
dans lequel l'application de diagnostic échographique dans le terminal portable (200) est configurée pour transmettre des informations de position du terminal portable (200) et des informations de partie diagnostiquée au serveur d'images échographiques (300),
dans lequel le serveur d'images échographiques (300) est configuré pour fournir des images échographiques de guidage d'une localisation de diagnostic et d'une partie diagnostiquée correspondant aux informations de position du terminal portable (200) et aux informations de partie diagnostiquée à l'application de diagnostic échographique, et
dans lequel l'application de diagnostic échographique dans le terminal portable (200) est configurée pour afficher l'image échographique avec une image échographique de guidage fournie à partir du serveur d'images échographiques (300).

2. Système de diagnostic échographique portable selon la revendication 1, dans lequel les informations de partie diagnostiquée sont définies par un utilisateur dans l'application de diagnostic échographique.

3. Système de diagnostic échographique portable selon la revendication 1, dans lequel le serveur d'images échographiques (300) est configuré pour recevoir des informations de recommandation d'une image échographique de guidage à recommander parmi des images échographiques de guidage pour chaque partie diagnostiquée à partir d'une application de diagnostic échographique dont l'utilisateur est un médecin généraliste, enregistrer un nombre de recommandations pour chaque image de guidage, et fournir une image échographique de guidage dont le nombre de recommandations est le plus élevé parmi les images échographiques de guidage de la localisation de diagnostic et de la partie diagnostiquée correspondantes à l'application de diagnostic échographique.

4. Système de diagnostic échographique portable selon la revendication 1, dans lequel le serveur d'images échographiques (300) est configuré pour fournir une pluralité d'images échographiques de guidage de la localisation de diagnostic et de la partie diagnostiquée correspondantes à chacune des applications de diagnostic échographique d'une pluralité d'utilisateurs, permettre aux utilisateurs de sélectionner l'image échographique de guidage à afficher avec l'image échographique à travers l'application de diagnostic échographique, enregistrer un nombre de sélections pour chaque image de guidage selon les sélections des utilisateurs, et fournir une image échographique de guidage dont le nombre de sélections est le plus élevé parmi les images échographiques de guidage de la localisation de diagnostic et de la partie diagnostiquée correspondantes à l'application de diagnostic échographique.

5. Système de diagnostic échographique portable selon la revendication 1, dans lequel le serveur d'images échographiques (300) est configuré pour enregistrer un rapport signal sur (RSB) pour chaque image échographique de guidage et fournir une image échographique de guidage dont le RSB est le plus élevé parmi les images échographiques de guidage de la localisation de diagnostic et de la partie diagnostiquée correspondantes à l'application de diagnostic échographique.

6. Système de diagnostic échographique portable selon la revendication 1, dans lequel l'application de diagnostic échographique est configurée pour transmettre des informations de genre ou des informations d'âge d'un objet à diagnostiquer au serveur d'images échographiques (300), et
dans lequel le serveur d'images échographiques (300) est configuré pour fournir une image échographique de guidage correspondant aux informations de genre ou aux informations d'âge parmi les images échographiques de guidage de la localisation de diagnostic et de la partie diagnostiquée correspondantes à l'application de diagnostic échographique.
